# EUROPEAN PATENT APPLICATION

(11) **EP 4 344 617 A1**
(43) Date of publication of application: **03.04.2024**
(21) Application number: 22197940.4
(22) Date of filing: 27.09.2022
(51) Int. Cl.: A61B 5/00

(54) **PAIN DETECTION**

(71) Applicant: Koninklijke Philips N.V., 5656 AG Eindhoven (NL)
(72) Inventor: RMAILE, Amir Hussein, Eindhoven (NL); JOHNSON, Mark Thomas, Eindhoven (NL); GERHARDT, Lutz Christian, Eindhoven (NL); SPELT, Hanne Adriana Alijda, Eindhoven (NL); KOOIJMAN, Gerben, EIndhoven (NL); PERRONE, Antonio Luigi, Eindhoven (NL)
(74) Representative: Philips Intellectual Property & Standards

(57) **Abstract**

The invention provides a method for obtaining a measure of user pain at home. The proposed concept is to measure the pain by utilizing sensor data obtained from force and/or motion sensors integrated in a personal care device, e.g. an oral care device, over the course of each of one or more personal care sessions of the user. Patterns in the sensor data, or changes in said patterns, can be used to infer pain information, such as an overall pain level, pain reaction events at certain points during the personal care session and/or at certain locations, or increased sensitivity at certain body areas . This provides a more objective and reliable estimation and monitoring of user oral pain that relying on patient (subjective) reporting of pain.

## Description

### FIELD OF THE INVENTION

This invention relates to a method and system for measuring user pain.

### BACKGROUND OF THE INVENTION

Following a dental treatment (e.g. tooth extraction, pocket cleaning, scaling, root canal, oral surgery), the dental practitioner has little or no way to monitor the healing process of the patient. The patient may believe that any experienced pain post-treatment is normal and thus not alert the dental practitioner, whereas such pain could instead be a sign of problems. As a result, problems may be missed, leading to medical complications or a worse clinical outcome. This is detrimental for the patient and also for the dental practitioner, and damages the dentist-patient relationship.

In a survey conducted by the applicant, in which 45 dental professionals were interviewed, a top highlighted concern was the absence of information about the progress of patient recovery after treatment.

The same problem also applies to monitoring patient status following a dental consultation at which the patient reported pain but no treatment was given. The dentist might ask the patient to monitor the condition and get back in touch in the event that the pain gets worse or does not abate. The patient might lose track of the progress of their pain, may acclimatize to the pain and so begin to ignore it, and thus not follow up with the dental practitioner to book a follow up appointment. This can lead to an initially minor problem becoming worse, with detrimental consequences for the patient's dental health.

A similar problem can likewise exist outside of the oral health domain, for instance related to skin health status in the area of dermatology. A dermatologist may provide treatment for skin, but has no way to track progress of the condition subsequently. Another example is the area of muscular joint pain, where improvement is hard to monitor without regular physical examinations.

A means to facilitate easier and more objective monitoring of a patient status post treatment or post consultation would therefore be of value.

### SUMMARY OF THE INVENTION

The invention is defined by the claims.

According to examples in accordance with an aspect of the invention, there is provided a method for determining a measure of user pain during one or more personal care sessions of a user using a personal care device. The method comprises: obtaining sensor data from one or more sensors integrated in a personal care device indicative of personal care action by the user using the device over the course of one or more personal care sessions; determining information indicative of user pain during each of the one or more personal care sessions based on analysis of patterns in the sensor data; and generating a data item indicative of the information indicative of user pain during each of the one or more personal care sessions.

In some embodiments, the sensors may include one or more force and/or motion sensors. In some embodiments, the sensors may include one or more operational signal sensors adapted to output an operational signal indicative of an operational parameter of the personal care device which correlates with physical interaction of the personal care device with bodily surfaces.

An aim of embodiments of this invention is to provide a means for semi-objectively monitoring patient pain levels at home. The proposed solution of the inventors is to efficiently utilize sensors (e.g. force and/or motion sensors) integrated in a personal care device (such as a powered toothbrush or shaver) to indirectly infer user pain levels. As will be explained below, inventors have realized that patterns in such sensor data can be used to infer user personal care behavior patterns over each personal care session and that these behavior patterns can be used to infer user experiences of pain. There are multiple different ways of doing this, as will become clear. For instance, it will be apparent that when a user feels pain, a reflexive motion response often follows, and thus it will be readily appreciated how the data of motion or force sensors might be used to detect this. Likewise, it will be recognized that applied pressure and patterns of scrubbing are typically different in anatomy areas which are painful compared to those which are not. Thus, the inventors have recognized that user pain can very efficiently and effectively be monitored through patterns in personal care device sensor data.

One application area is in the domain of oral care. In some embodiments, the personal care device is an oral care device, the aforementioned personal care action is cleaning action, and the personal care session is an oral cleaning session. In further embodiments, the personal care device may be a skin care device, e.g. a light therapy device, and the personal care action is skin care action and the personal care session is a skin care session. In further embodiments, the personal care device is a grooming device, e.g. a shaver, and the personal care action is a grooming action (e.g. shaving), and the personal care session is a grooming session (e.g. shaving). In further embodiments, the personal care device is a muscle/joint care device (e.g. a massage device). In each case, the personal care device is a device for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through monitoring patterns in the sensor data.

As noted, the one or more sensors may include one or more force and/or motion sensors, and/or one or more operational signal sensors adapted to output an operational signal indicative of an operational parameter of the personal care device which correlates with physical interaction of the personal care device with bodily surfaces. An example of an operational parameter is an electrical drive signal associated with an actuation device which actuates a portion of the personal care device designed for making contact with the user in use, e.g. a drive train mechanism which drives oscillation of a body-contacting portion of a powered toothbrush or shaver, or a motor mechanism which drives a body-contacting portion of a massage device. The electrical characteristics of the drive signal can change as a function of applied pressure since applied pressure exerts more physical resistance on the actuation mechanism. Another example of an operational parameter is jet flow of an oral irrigation device or powered flossing device.

It is noted that integrated force and/or motion sensors might be sensors that are also utilized for other sensing purposes, such as monitoring user brushing behavior (in the case of oral care) in order to provide feedback and advice on brushing coverage and brushing technique. Thus, in practice, the sensors that are used according to the proposed inventive concepts might already be integrated in the personal care device. The inventive concept could even be enabled in some embodiments purely through a software update, utilizing existing hardware in the personal care device.

In some embodiments, the information indicative of user pain during each of the one or more personal care sessions is determined further based on reference sensor pattern information associated with user pain or user comfort. In a simple example, this could be a reference level of pressure which the user normally applies when they are not in pain. This might be collected before a treatment for example, to provide a baseline against which future sensor data patterns can be compared.

In some embodiments, the reference sensor pattern information may include one or more characteristic pattern features which are each indicative of either a user pain reaction at a given moment during a personal care session, or user comfort during a given period of a cleaning session. Here, it is proposed that the reference information include certain standardized waveform features or patterns which are indicative of a user pain response or user comfort. For example, a sudden spike in motion in a direction away from the tooth surface might be indicative of a pain reaction. A continuous high amplitude brushing motion might be indicative of comfort.

In some embodiments, the information indicative of user pain comprises detecting one or more of the characteristic pattern features within at least one sensor signal waveform included in the sensor data. In other words, certain signature waveform features, such as waveform patterns, shapes, or morphologies, are detected indicative of a pain event or indicative of comfort.

In some embodiments, the determining of the information indicative of user pain comprises determining information indicative of user pain as a function of time over the course of each of the one or more personal care sessions. For example, a pain signal may be determined, indicative of pain level as a function of time.

Alternatively, the information indicative of user pain could include one or more indicators or metrics which are indicative of pain over the whole of or part of the session, e.g. a statistical measure of pain or an average measure of pain, or a maximum or minimum level of pain, etc.

In some embodiments, the information indicative of user pain is determined based on detecting one or more inflections in at least one sensor signal waveform included in the sensor data, the one or more inflections being indicative of user pain response events during the given personal care session. Here the characteristic pattern feature is an inflection in the signal.

Additionally or alternatively, in some embodiments, the sensor data includes a pressure sensor waveform indicative of application pressure of a portion of the personal care device to body or anatomy surfaces during use (e.g. oral surfaces in the case of oral care or skin surfaces in the case of skin care or grooming), and wherein the information indicative of user pain is determined based on the pressure signal. The pressure signal itself could in some instances be used as a direct proxy for pain, as a user will typically press less hard when they are in pain compared to when they feel comfort.

Additionally or alternatively, the pain information could be inferred from certain characteristic pattern features in the pressure sensor signal.

For example, in some embodiments, the information indicative of user pain is determined based on detecting valleys in the pressure sensor waveform, indicative of a drop in applied pressure during a sub-interval of a given personal care session. Here the characteristic pattern feature is a valley in the signal. A drop in pressure is an indication that the user experiences pain.

In some embodiments, the reference sensor data pattern information comprises reference sensor data acquired for the same user at a time or at a location of the body/anatomy (e.g. location in the mouth for oral care or a location on the skin for skin care or grooming) known to be associated with a baseline pain state, and optionally wherein the baseline pain state is a low pain state. For example, this could be data acquired pre-treatment and associated with a low pain state, or data acquired from another area in the mouth or another area on the skin to an affected area (e.g. an opposite side of the mouth, or opposite side of the body), associated with a low pain state.

In some embodiments, the sensor data includes a motion sensor signal, and wherein the information indicative of user pain is determined based on detecting inflections in motion, indicative of user reaction to locally experienced pain.

Here the characteristic pattern features include upward inflections in a motion sensor signal. The motion sensor could include an inertial sensor such as an accelerometer.

In some embodiments, the sensor data includes a motion sensor signal and wherein the information indicative of user pain is determined based on detecting in the motion sensor signal periods of alternating motion, indicative of a scrubbing action by the user, and wherein an amplitude or time duration of the periods of alternating motion are used to infer a level of patient comfort.

In some embodiments, the sensor data includes position sensor data, and wherein the method comprises determining information indicative of user pain as a function of location, i.e. as a function of location on/in the anatomy to which the device is applied. In some embodiments, if the personal care device is an oral care device, the sensor data may include position sensor data, and wherein the method comprises determining information indicative of user pain as a function of location in the mouth.

In some embodiments, the information indicative of user pain during each of the one or more personal care sessions is determined based on reference sensor pattern information which includes one or more characteristic pattern features which are each indicative of either a user pain reaction at a given moment during a personal care session, or user comfort during a given period of a personal care session. In some embodiments, the determining the measure indicative of user pain as a function of location is based on using the position sensor data to correlate each of the detected characteristic pattern features to a corresponding position, e.g. a corresponding position on the body, e.g. a corresponding position on/in the anatomy to which the personal care device is for being applied. In some embodiments, the method further comprises generating a pain map indicative of user pain as a function of location and optionally communicating the generated pain map to a user interface for display on the user interface

In some embodiments, the sensors in the personal care device may be pre-existing sensors already used for other functions. In other words, the method may further comprise processing the sensor data for the purpose of performing one or more other operational functions of the device. Thus, there is structural efficiency in re-using the same sensors.

The invention can also be embodied in software. Thus, another aspect of the invention is a computer program product comprising computer program code means configured, when run on a processor, to cause the processor to perform the method in accordance with embodiment described in this document, or in accordance with any claim of this application.

The invention can also be embodied in hardware form. Thus, another aspect of the invention is a processing device, comprising: an input/output; and one or more processors. The one or more processors are configured to: receive at the input/output sensor data generated by one or more sensors integrated in an personal care device indicative of personal care action by the user using the device over the course of one or more personal care sessions; determine information indicative of user pain during each of the one or more personal care sessions based on based on analysis of patterns in the sensor data, and optionally based on reference sensor pattern information associated with user pain or user comfort; and generate a data item indicative of the determined information indicative of user pain during each of the one or more personal care sessions, and preferably routing the data item to the input/output for export from the processing device.

This processing device could be integrated in the personal care device itself, and may include a communication module for communicating the information indicative of user pain to an external device, e.g. a memory or a computing device.

The processing device could be a processing device of an external computing device, e.g. a mobile computing device, and wherein the sensor data is received via communication with the personal care device or with a datastore with which the personal care device is in communication, e.g. a cloud based datastore.

The processing device could be a cloud-based processing device.

Another aspect of the invention is a system comprising: a personal care device comprising: one or more sensors configured to generate sensor data indicative of personal care action by the user using the device over the course of one or more personal care sessions; and a processing device as outlined above, or in accordance with any embodiment described in this document, and arranged to receive the generated sensor data as an input at the input/output. In one set of embodiments, the personal care device is an oral care device.

In some embodiments, the processing device may be integrated in the personal care device.

In some embodiments, the processing device may be comprised by a computing device external to the personal care device, the computing device communicatively coupled with the personal care device or with a datastore with which the personal care device is communicatively coupled.

In some embodiments, the processing device may be a cloud-based processing device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of components of an example processing device and system according to one or more embodiments of the invention;
Fig. 3 illustrates recorded brushing action sensor data in the form of a brushing pressure signal which might be used to infer user pain from a valley in the brushing pressure signal;
Fig. 4 illustrates recorded brushing action sensor data in the form of motion data in a direction away from a surface of a tooth which might be used to infer user pain from an upward inflection, or spike, in the signal;
Fig. 5 illustrates recorded brushing action sensor data in the form of motion data in a direction in-plane with a surface of a tooth, and illustrates how user pain might be inferred from a reduced amplitude in an alternating scrubbing motion pattern; and
Figs. 6 and 7 illustrate comparative example plots of average brushing pressure over a monitoring period which includes a period prior to a treatment and following a treatment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for obtaining a measure of user pain in one or more anatomical areas or regions at home. The proposed concept is to measure the pain by utilizing sensor data obtained from usage sensors (e.g. force and/or motion sensors) integrated in a personal care device over the course of each of one or more personal care sessions of the user. Patterns in the sensor data, or changes in said patterns, can be used to infer pain information, such as an overall pain level, pain reaction events at certain points during the personal care session and/or at certain locations, or increased sensitivity in certain anatomical areas. This provides a more objective and reliable estimation and monitoring of user personal care pain than relying on patient (subjective) reporting of pain.

The concept is based on the principle that systematic use of a personal care device is something that a user routinely does at regular and reliable time points, and that its normal use typically entails application of a systematic series of physical stimuli to an anatomy or different areas of an anatomy. This therefore provides an efficient way of measuring pain information associated with the relevant anatomy, and using data captured from sensors associated with the personal care device.

By way of example, one application area is for use with oral care devices, for example used for oral cleaning. Since tooth cleaning is something that a person usually already does twice a day, the monitoring approach can very easily and efficiently be integrated in a user's normal daily routine, making compliance likely very high. Also, force/motion sensors might already be integrated in the oral care device, for other functional purposes, and thus the additional hardware requirements are minimal or none at all.

One advantage of embodiments of the invention is enabling a clinical practitioner (e.g. dental practitioner, dermatologist, etc.) to stay informed about the progress of patient status even while the patient is at home. This leads to several advantages. It leads to improved patient experience by providing peace of mind that their healing is being monitored by their practitioner. Staff experience is improved by providing the practitioner an insight into how the patient condition is progressing (or regressing). Clinical efficiency is also improved by optimizing practitioner time: for example, scheduling of a follow-up appointment can be deferred until pain data indicate that a problem may be present that requires examination. Another benefit is improved health outcomes. For example, post treatment monitoring of healing increases the odds of a successful treatment.

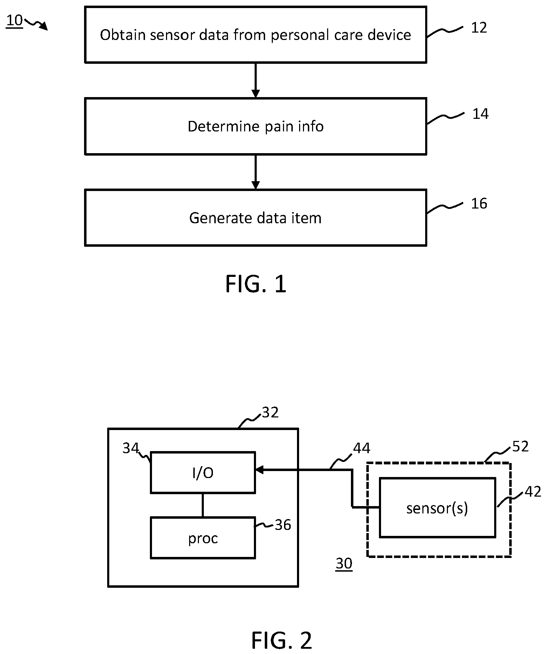

Fig. 1 outlines in block diagram form steps of an example computer implemented method 10 according to one or more embodiments. The steps will be recited in summary, before being explained further in the form of example embodiments.

The method 10 is for determining a measure of user pain during one or more personal care sessions of a user using a personal care device.

The method 10 comprises obtaining 12 sensor data from one or more sensors integrated in a personal care device indicative of personal care action by the user using the device over the course of one or more personal care sessions.

The method 10 further comprises determining 14 information indicative of user pain during each of the one or more personal care sessions based on analysis of patterns in the sensor data.

The method 10 further comprises generating 16 a data item indicative of the information indicative of user pain during each of the one or more personal care sessions.

The method may further comprise generating a report based on the data item and transmitting the report via a communication channel to a remote computer, for example a computer accessible to a practitioner such as a dental practitioner. For example, the report may be uploaded to a server, for example a cloud-based server or other server which is accessible to the practitioner. This way, the practitioner can be informed as to the pain status of the patient

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system does not have to comprise all of the illustrated hardware components; it may just comprise a subset of them.

The processing device comprises one or more processors 36 configured to perform a method in accordance with that outlined above, or in accordance with any embodiment described in this document or any claim of this application. In the illustrated example, the processing device further comprises an input/output 34 or communication interface. In the illustrated example of Fig. 2, the system 30 further comprises one or more sensors for measuring sensing data indicative of personal care action by the user using the device over the course of one or more personal care sessions. The one or more sensors are integrated in a personal care device 52 for use by the user. The personal care device may be part of the system 30 in some embodiments.

The one or more sensors may include one or more force and/or motion sensors for sensing motion of the device and/or forces exerted on or by the device. Additionally or alternatively, data may be used from one or more operational signal sensors integrated in the oral care device adapted to output an operational signal indicative of an operational parameter of the personal care device which correlates with physical interaction of the personal care device with bodily surfaces. An example of an operational parameter is an electrical drive signal associated with an actuation device which actuates a portion of the personal care device, e.g. a drive train mechanism which drives oscillation of a powered toothbrush or shaver, or a motor mechanism which drives a massage device. The electrical characteristics of the drive signal can change as a function of applied pressure since applied pressure exerts more physical resistance on the actuation mechanism. Another example of an operational parameter is jet flow of an oral irrigation device or powered flossing device.

In some embodiments, the processing device 32 is integrated in the personal care device. In further embodiments, the processing device 32 may be comprised by a computing device external to the personal care device, the computing device communicatively coupled with the personal care device or with a datastore with which the personal care device is communicatively coupled. In further embodiments, the processing device 32 may be a cloud-based processing device.

To illustrate and exemplify the inventive concepts, examples are presented below with reference to an oral care device, for which the aforementioned personal care action is cleaning action, and the personal care session is an oral cleaning session. However, it will be recognized that the principles outlined are easily applicable to other types of personal care device, such as, by way of example, a skin care device, e.g. a light therapy device, a grooming device, e.g. a shaver, or a muscle/joint care device (e.g. a massage device). With any personal care device, the device is typically designed for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through monitoring patterns in the sensor data. Thus it can be seen how the principles of the general inventive concept outlined above, and described below, can be used in any personal care device. Hence, reference in the below-described embodiments to an oral care device may be replaced by reference to any other personal care device without substantive change to the inventive principles. Likewise, reference to cleaning action can be replaced by reference to personal care action and reference to a cleaning session can be replaced by reference to a personal care session.

In the case that the personal care device is an oral care device, the oral care device may be a powered toothbrush in some embodiments. The toothbrush may have a brush head carrying an array of cleaning elements such as bristles. Integrated force and/or motion sensors may include a pressure sensor for sensing an application pressure of the cleaning elements of the brush head to teeth during operation. Such a pressure sensor could be integrated in the brush head itself, but more usually would be integrated in the handle portion of the toothbrush, to which the brush head is attached. The brush head may be removably attached to the handle portion to enable replacement. Application pressure to teeth can be sensed e.g. via a cantilever pressure sensing approach.

Integrated force and/or motion sensors may include a motion sensor for sensing motion of the personal care device. The motion sensor may comprise or take the form of an inertial measurement unit (IMU) comprising typically one or more accelerometers, for example a tri-axial accelerometer arrangement.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

There are different approaches to performing the analysis of patterns in the sensor data in order to determine the information indicative of user pain.

According to one approach, the obtained sensor data includes a pressure sensor waveform indicative of application pressure of a portion of a personal care device to surfaces during use, and wherein the information indicative of user pain is determined based on the pressure signal. For example, if the personal care device is an oral care device, e.g. a powered toothbrush device, the pressure sensor waveform may be indicative of application pressure of the brush head cleaning elements (e.g. bristles) to tooth/oral surfaces. Such pressure sensor data provides information indicative of cleaning action by the user using the device over the course of one or more oral cleaning sessions because it relates to how hard the user is pressing the cleaning elements onto the oral/dental surfaces during the cleaning session. Likewise, if the personal care device is grooming device, e.g. a shaver, the pressure sensor waveform may be indicative of application pressure of the shaver cutting area to the skin of the user during use. Such pressure sensor data provides information indicative of shaving action by the user over the course of one or more shaving sessions. Similar principles apply for other types of personal care device.

Application pressure sensor data is useful for inferring information indicative of user pain during a personal care session because it is known that the application pressure will be inversely related to the level of pain experienced (e.g. the brushing pressure reduces as the pain increases). Thus, analysis of the pattern of pressure over the course of a single personal care cycle, or multiple cycles, can provide information about user pain experience. For example, in a most simple embodiment, the observation of the application pressure, e.g. brushing pressure, during the entire personal care cycle, e.g. cleaning cycle, will show a pattern whereby differences in spread of application pressures as a function of time or location (for example compared to the baseline measurements) will provide an indication of pain: namely if unexpected drop in pressure is seen in the pattern (where normally a fairly constant pressure would be observed), this may be an indication of a pain-causing abnormality in the lower-pressure area. Even more simply than this, a standard threshold pressure could be set, and wherein any drop in applied pressure below this threshold over a section of the sampled signal is taken as an indication of a pain experience in the location of the anatomy (e.g. mouth) to which that signal section corresponds.

In relation to this set of embodiments, as a general approach, it could be stated that the information indicative of user pain during each of the one or more personal care sessions can be determined further based on reference sensor pattern information associated with user pain or user comfort. The reference sensor pattern information could include a reference pattern of baseline brushing pressure as a function of time (or location) over the course of a personal care cycle, personalized to the user, and wherein pain in a personal care session is detected by comparing this reference baseline pattern to a pattern measured in the new personal care session. More simply, the reference sensor pattern information may include one or more characteristic pattern features in the sensor signal waveform which are each indicative of either a user pain reaction at a given moment during a personal care session, or user comfort during a given period of a personal care session. For the pressure sensor signal in particular, the relevant characteristic pattern feature would be a valley, or negative spike, in the pressure sensor waveform, indicative of a drop in applied pressure during a sub-interval of a given personal care session. For example, a valley in the signal in which the pressure falls below a pre-defined threshold baseline pressure could indicate a pain event for the sub-interval of the personal care session for which the pressure remains below that threshold. The threshold might be set according to a reference baseline pressure, for example acquired pre-treatment or otherwise during a period or at an anatomy location associated with low or zero pain.

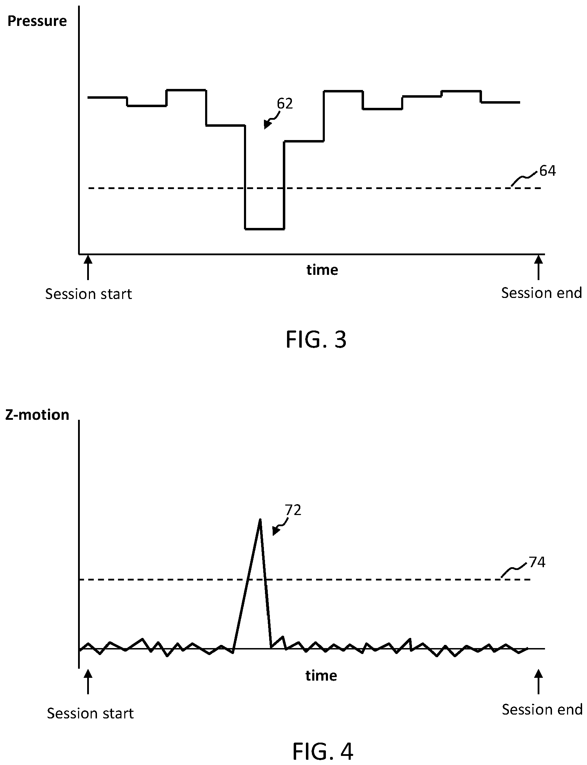

Fig. 3 illustrates an example in the context of an oral care device for performing a cleaning function. Fig. 3 illustrates an example brushing pressure signal as a function of time. A valley in the signal is indicated by arrow 62. During the sub-interval of time spanned by this valley, the applied pressure is below a defined threshold 64 and so it is determined that a pain event occurred during the sub-interval of time spanned by the valley. A valley 62 of depth lower than the threshold 64 represents a characteristic pattern feature whose detection is indicative of pain being experienced for the period of time spanned by the valley.

Optionally, in some embodiments, the pressure signal as a function of time or location in the mouth could be converted into a signal indicative of user pain as a function of time or location over the course of each of the one or more cleaning sessions. In a simple example, the pressure signal could be used directly as a proxy for pain, since it is expected to be inversely correlated with experienced pain.

As mentioned above, the brushing pressure signal would be rendered much more specific by addition of location information. The combination of a location sensor and a pressure sensor enables each pain event to be associated with an oral location. This allows a dentist or the user to know whether a pressure drop coincides with the position of a particular dental issue such as a post-treatment wound, whereby the signal can be attributed to pain level with greater certainty.

Thus, in some embodiments, the obtained sensor data may include position sensor data, and wherein the method comprises determining information indicative of user pain as a function of location in the mouth. The determining the measure indicative of user pain as a function of location in the mouth may be based on using the position sensor data to correlate each of the detected valleys in the pressure signal to a corresponding position in the mouth. The method may furthermore comprise generating a pain map indicative of user pain as a function of location in the mouth and optionally communicating the generated pain map to a user interface for display on a user interface.

Furthermore, it is noted that the utility of a position sensor to correlate pain events with location in the mouth is not confined to embodiments which use a pressure sensor. Regardless of which particular force and/or motion sensor data is used to infer the pain information, the additional use of a location sensor allows characteristic pattern features associated with pain which are detected in the force/motion sensor signal to be correlated with a location in the mouth. Generation of a pain map, as suggested above, is also compatible more widely with any of the other described embodiments in this document.

In accordance with a further approach (which could be either combined with or used in place of the pressure-sensor approach already described), one or more motion sensing means can be used to sense motion of the personal care device. In this case, the sensor signal may show a sudden (impulse) movement of the personal care device as an acute pain is felt. The movement might be e.g. perpendicular to (e.g., away from) the anatomy surface (e.g. tooth surface), but sudden movement in any direction is possible following a feeling of pain. Here, the level of pain can be inferred as increasing as the motion impulse increases.

Thus, according to this approach, the sensor data may include a motion sensor signal, and wherein the information indicative of user pain is determined based on detecting inflections in motion, indicative of user reaction to locally experienced pain.

Thus this approach is also based on detecting one or more characteristic pattern features in the sensor signal waveform indicative of either a user pain reaction at a given moment during a personal care session, or user comfort during a given period of a personal care session. In this approach, the characteristic waveform pattern feature is an inflection or spike or impulse in the signal.

The motion sensor could include an inertial sensor such as an accelerometer for example.

Fig. 4 illustrates an example motion sensor signal as a function of time over the course of a single personal care session. The motion sensor in this case is sensitive at least to motion in a direction perpendicular (e.g. away from) the relevant anatomy surfaces (e.g. tooth surfaces) during use of the personal care device (denoted as the z-direction in Fig. 4). An upward inflection or spike or fluctuation in the signal is indicated at arrow 72. The amplitude or height of the inflection or spike or fluctuation in the signal exceeds a threshold motion level 74, which might in this instance correspond to a speed of motion or a rate of acceleration. If an inflection in the signal exceeds the pre-defined threshold 74, it may be classified as corresponding to a pain event.

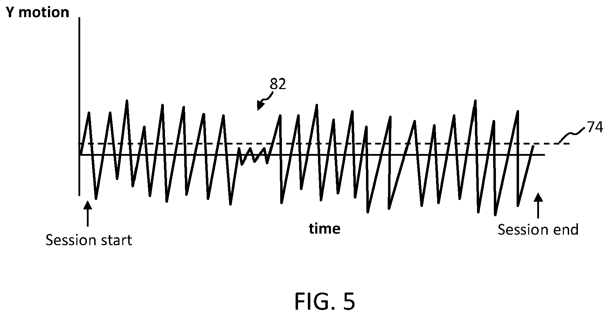

According to a further approach (which may be either combined with or used instead of any of the other approaches in this document), a motion sensor may be used which is operable to detect motion during use of the personal care device. The patterns in this signal are representative of scrubbing action by a user, which manifests as an alternating pattern in the signal waveform. Fig. 5 shows an example. In this example, motion in a direction in-plane with a tooth surface (y-direction) is shown by way of illustration in the graph of Fig. 5. However, scrubbing motion could be sensed with a motion sensor sensing motion in any direction, e.g. simply a tri-axial accelerometer operable to sense motion in any direction.

At locations where pain is experienced, it is to be expected that amplitude of scrubbing motion would decrease compared to that in areas where no pain is felt. Thus here, the sensor data includes a motion sensor signal and wherein the information indicative of user pain is determined based on detecting in the motion sensor signal periods of alternating motion, indicative of a scrubbing action by the user, and wherein an amplitude or time duration of the periods of alternating motion are used to infer a level of patient comfort.

In particular, pain may be detected by detecting a characteristic waveform feature in the form of a section of an alternating signal having an amplitude which falls below a threshold 74. The threshold might be set in some examples as a defined proportion of an average amplitude of the signal over the whole of the personal care session, for example half of the average amplitude. A sub-section of the signal during which the amplitude of a detected alternating motion signal is below the defined threshold may be classified as corresponding to pain events. Again, use of a location sensor in addition may allow these sub-sections of the signal to be correlated with particular locations of the anatomy, e.g. in the mouth, e.g. particular dental sites.

For example, the acquired sensor data can include position sensor data, and wherein the method comprises determining information indicative of user pain as a function of location on/in the anatomy, e.g. in the mouth. User pain as a function of location may be determined based on using the position sensor data to correlate each of the detected signal periods of lower-amplitude alternating motion to a corresponding position on/in the anatomy, e.g. in the mouth.

In addition to or instead of amplitude of the scrubbing motion, also frequency of scrubbing can be used to detect areas of higher pain. In particular, slow careful movements or scrubbing (at that location) are to be expected in areas of higher pain. Thus, in some embodiments, determining information indicative of user pain comprises determining a frequency of an alternating motion signal along a direction parallel with application surfaces (e.g. tooth surfaces), identifying continuous motion signal sections for which frequency is below a threshold frequency, and classifying those sections as correspond to pain events. Again, here the addition of a location sensor will improve specificity.

In addition to or instead of amplitude and frequency of the scrubbing motion, also the duration of time spent applying the device to a particular area, e.g. duration of time spent cleaning a particular tooth or dental area, could be used to infer a level of pain experienced at that area. Here, the analysis of the sensor signal might comprise first identifying in a force and/or motion sensor signal a plurality of different signal sections corresponding to periods of time spent cleaning different areas, such as different dental areas, such as different teeth. In some cases, this could be done though correlating different signal sections with the output of a motion sensor. In other cases, it could be done through identifying pattern sections separated by breaks or pauses in the signal as the user moves to the next signal. For example for a pressure signal, the pauses might have a recognizable signature or fingerprint, such as being signal periods of a duration being below a threshold duration, and wherein for instance pressure or motion amplitude is below a certain threshold. Once the different signal sections have been identified, a time duration of each one can be determined, and from this it can be determined how long the user spent treating each area, e.g. cleaning each tooth. A short time may indicate a higher pain. A short time combined also with presence during that signal section of one of the characteristic signal waveform features discussed, such as a lower amplitude alternating motion, or a spike in out-of-plane motion, or a dip in pressure, might be used to indicate pain.

Although the above example was described with reference to an example signal corresponding to in-plane motion, in practice the detected alternating motion can be in any direction.

Above have been described example techniques for determining information indicative of user pain based on identifying certain characteristic pattern features in the sensor signal(s). A further approach, which could be used in combination with or instead of the already described approaches, is to compare all or part of an acquired force and/or motion sensor signal waveform with a personalized reference waveform, acquired at a time or over an area which is known to be associated with low pain. In other words, reference sensor data pattern information is acquired in advance for the user at a time or in a location of the anatomy (e.g. location in the mouth) known to be associated with a baseline pain state, and preferably wherein the baseline pain state is a low pain state. For example, it might be acquired prior to a treatment (e.g. a dental treatment), or prior to emergence of a new complaint. Then, the information indicative of user pain during each of the one or more personal care sessions is determined by comparing a sensor signal for a new personal care session with the reference sensor pattern information associated with user pain or user comfort.

An illustrative example might be implemented as follows, in this case with reference to oral care with an oral care device. First, prior to a dental treatment, or during a calibration period unconnected with any treatment, normal brushing behavior of the user is monitored over a period of time, for instance 3-7 days using the force and/or motion sensors integrated in the oral care device, e.g. powered toothbrush. From this, an average signal waveform as a function of time over a cleaning session may be determined from the acquired data for each of the one or more force and/or motion sensors. From this, a single characteristic or representative value might be obtained. For instance, an average applied pressure. Alternatively, the whole average waveform may be obtained.

After the calibration period, for example after a dental treatment, the force and/or motion sensor data can be monitored in the same way as before, and the recorded signal waveform for each cleaning session can be compared against the reference acquired in the calibration period. Either the whole waveform can be compared against the reference, to therefore detect signal sections which deviate significantly (which could then optionally be correlated to particular mouth locations), or the single characteristic or representative value is compared. Significant deviations may be indicative of a change in pain state.

In other words, any immediate change from the pretreatment pattern is indicative of pain. If this change remains consistent, then pain is still there. Duration (optionally spread and intensity) of pain can now be extracted, and, depending on the procedure - the dental practitioner is provided insights as to whether a follow up with the patient is needed (if pain persists longer than normal) or if progress is normal.

Once the patient returns to the pattern recorded in the calibration period (e.g. the pre-treatment pattern), this means that the pain has gone. The dental practitioner could even use this information for example to cancel a planned follow up appointment as no longer needed.

Exactly the same principle could be applied for other personal care devices, e.g. skin care device in the context of a skin care treatment, a massage device in the context of a muscle care treatment, etc.

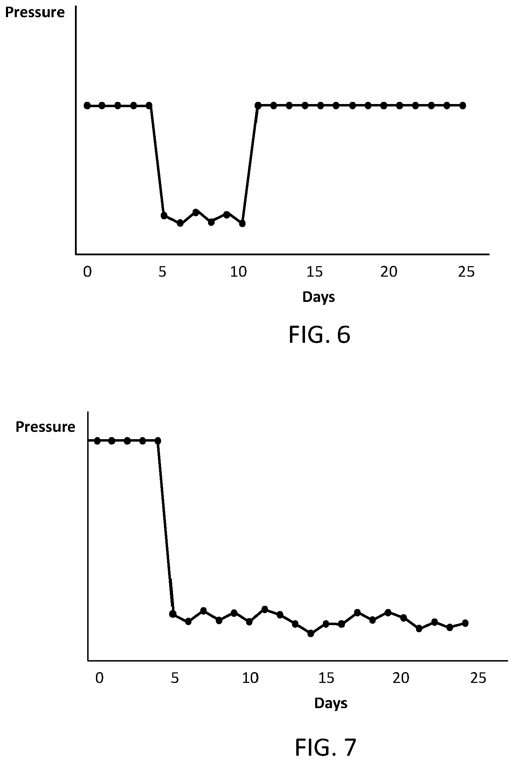

To illustrate visually, Fig. 6 schematically illustrates an example period of 25 days over which oral cleaning sensing data is acquired. The period from day 1 to day 4 is the calibration period. For simplicity, in this example, a single representative value of average brushing pressure was extracted from the brushing pressure waveforms for the cleaning sessions each day. These are plotted as data points on the graph. At day 5, the patient undergoes a dental treatment that leaves a wound at a dental site. It can be seen that average brushing pressure immediately declines. This can be taken as indicative of user pain. The brushing pressure varies day-by-day over an approximately 6 day period, while remaining at a level significantly below the reference or baseline level measured during the calibration period in days 1-4. Then, around day 10 or 11, the average pressure recovers to approximately the level prior to the treatment. This indicates that the pain has resolved and the user has returned to normal. It can be inferred that the wound left by the treatment has healed.

Fig. 7 illustrates a comparative example in which the average pressure instead remains around the significantly reduced level following the treatment, and does not recover. This indicates that pain has not resolved, even after 20 days following the treatment. This might indicate that the wound is failing to heal normally.

It is noted that in a more basic implementation, the calibration phase in which the reference waveform or reference value is acquired may not be needed. Instead the reference might be acquired from measuring sensor data at an area known to be associated with a baseline low pain state, such as a different area of the anatomy (e.g. opposite side of the mouth) to the area that was treated or to an area affected by a pathology.

It is further noted that although the examples discussed above are presented in terms of monitoring before and after a treatment, the described approach is applicable in any scenario, whether there is a treatment or not. Pain can be associated with a pathology which is unconnected with any treatment, and a change in the measured sensor data patterns compared to a reference time or a reference area can indicate pain, and thus indicate a change in health status.

As has been noted above, in accordance with any embodiment of the invention, position sensor data indicative of a location or position of an operative part of the personal care device can be acquired and used to register force and/or motion sensor waveforms to position relative to the body/anatomy, e.g. position in the mouth in the case of an oral care device. Using the combination of location and pressure/motion sensors allows the generation of pain maps showing the relative pain spread and level of pain. Monitoring such pain maps over time will enable improved diagnosis.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing arrangement includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for determining a measure of user pain during one or more personal care sessions of a user using a personal care device, the method comprising:
obtaining sensor data from one or more sensors integrated in a personal care device indicative of personal care action by the user using the device over the course of one or more personal care sessions;
determining information indicative of user pain during each of the one or more personal care sessions based on analysis of patterns in the sensor data; and
generating a data item indicative of the information indicative of user pain during each of the one or more personal care sessions.

2. The method of claim 1, wherein the one or more sensors include:
one or more force and/or motion sensors; and/or
one or more operational signal sensors adapted to output an operational signal indicative of an operational parameter of the personal care device which correlates with physical interaction of the personal care device with bodily surfaces.

3. The method of claim 1 or 2, wherein the information indicative of user pain during each of the one or more personal care sessions is determined further based on reference sensor pattern information associated with user pain or user comfort.

4. The method of claim 3, wherein the reference sensor pattern information includes one or more characteristic pattern features which are each indicative of either a user pain reaction at a given moment during a personal care session, or user comfort during a given period of a personal care session.

5. The method of claim 4, wherein determining the information indicative of user pain comprises detecting one or more of the characteristic pattern features within at least one sensor signal waveform included in the sensor data.

6. The method of any of claims 1-5, wherein the information indicative of user pain is determined based on detecting one or more inflections in at least one sensor signal waveform included in the sensor data, the one or more inflections being indicative of user pain response events during the given personal care session.

7. The method of any of claims 1-6, wherein the sensor data includes a pressure sensor waveform indicative of application pressure of a portion of the personal care device to surfaces during use, and wherein the information indicative of user pain is determined based on the pressure signal, and
optionally wherein the information indicative of user pain is determined based on detecting valleys in the pressure sensor waveform, indicative of a drop in applied pressure during a sub-interval of a given personal care session.

8. The method of any of claims 1-7, wherein the reference sensor data pattern information comprises reference sensor data acquired for the same user at a time or at a bodily location known to be associated with a baseline pain state, and optionally wherein the baseline pain state is a low pain state.

9. The method of any of claims 1-8, wherein the sensor data includes a motion sensor signal, and wherein the information indicative of user pain is determined based on detecting inflections in motion, indicative of user reaction to locally experienced pain.

10. The method of any of claims 1-9, wherein the sensor data includes a motion sensor signal and wherein the information indicative of user pain is determined based on detecting in the motion sensor signal periods of alternating motion, indicative of a scrubbing action by the user, and wherein an amplitude or time duration of the periods of alternating motion are used to infer a level of patient comfort.

11. The method of any of claims 1-10, wherein the sensor data includes position sensor data, and wherein the method comprises determining information indicative of user pain as a function of location.

12. The method of claim 11,
wherein the information indicative of user pain during each of the one or more personal care sessions is determined based on reference sensor pattern information which includes one or more characteristic pattern features which are each indicative of either a user pain reaction at a given moment during a personal care session, or user comfort during a given period of a personal care session; and
wherein determining the measure indicative of user pain as a function of location is based on using the position sensor data to correlate each of the detected characteristic pattern features to a corresponding position.

13. The method of claim 11 or 12, wherein the method comprises generating a pain map indicative of user pain as a function of location and optionally communicating the generated pain map to a user interface for display on the user interface

14. The method of any of claims 1-13, wherein the personal care device is an oral care device, the personal care action is cleaning action, and the personal care session is an oral cleaning session.

15. A computer program product comprising code means configured, when run on a processor, to cause the processor to perform the method in accordance with any of claims 1-14.

16. A processing device, comprising:
an input/output; and
one or more processors, configured to:
receive at the input/output sensor data generated by one or more sensors integrated in a personal care device indicative of personal care action by the user using the device over the course of one or more personal care sessions;
determine information indicative of user pain during each of the one or more personal care sessions based on based on analysis of patterns in the sensor data, and optionally based on reference sensor pattern information associated with user pain or user comfort; and
generate a data item indicative of the determined information indicative of user pain during each of the one or more personal care sessions, and preferably routing the data item to the input/output for export from the processing device.

17. A system comprising:
a personal care device comprising: one or more force or motion sensors configured to generate sensor data indicative of personal care action by the user using the device over the course of one or more personal care sessions; and
a processing device in accordance with claim 16, arranged to receive the generated sensor data as an input at the input/output.
